(19) Europäisches Patentamt
European Patent Office
Office européen des brevets

(11) Veröffentlichungsnummer: **0 039 830**
**A1**

# (12) EUROPÄISCHE PATENTANMELDUNG

(21) Anmeldenummer: **81103211.9**

(22) Anmeldetag: **29.04.81**

(51) Int. Cl.³: **C 07 B 1/00**
B 01 J 31/28, C 07 D 311/72
C 07 D 307/32, C 07 D 307/60

(30) Priorität: **14.05.80 DE 3018388**

(43) Veröffentlichungstag der Anmeldung:
**18.11.81 Patentblatt 81/46**

(84) Benannte Vertragsstaaten:
**CH DE FR GB LI**

(71) Anmelder: **BASF Aktiengesellschaft**
**Carl-Bosch-Strasse 38**
**D-6700 Ludwigshafen(DE)**

(72) Erfinder: **Vogel, Friedrich, Dr.**
**Im Kleinen Letten 3**
**D-6706 Wachenheim(DE)**

(72) Erfinder: **Paust, Joachim, Dr.**
**Ringstrasse 3**
**D-6701 Neuhofen(DE)**

(72) Erfinder: **Nuerrenbach, Axel, Dr.**
**Koenigsberger Strasse 7**
**D-6718 Gruenstadt(DE)**

## (54) Verfahren zur Herstellung von optisch aktiven Verbindungen und deren Verwendung zur Herstellung von Vitamin E.

(57) Herstellung von optisch aktiven Verbindungen der Formel I

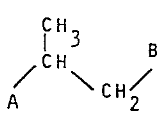

1

in der A eine $-CH_2OR^1$ Gruppe, wobei $R^1$ für Wasserstoff, niedermolekulares Alkyl, Aryl oder niedermolekulares Acyl steht, ferner eine gegebenenfalls acetalisierte oder acylierte Formylgruppe oder eine Carboxylgruppe bedeutet, B eine gegebenenfalls acetalisierte oder acylierte Formylgruppe oder eine Gruppe der Formel $-COOR^2$ bedeutet, in der $R^2$ für einen niedermolekularen Alkylrest steht, und wobei A und B stets verschieden sind und wobei ferner A und B zusammen die Gruppe $CH_2$-O-CO- bedeuten können, durch Hydrieren von Verbindungen der Formel II

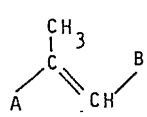

11

in Gegenwart eines in der Reaktionsmischung löslichen Komplexkatalysators hydriert, der aus einer Rhodium- oder Rutheniumverbindung und einem chiralen Phosphin- oder Arsin-Liganden gebildet ist.

Man erzielt gute optische Ausbeuten.

So erhältlicher Verbindungen eignen sich zur Herstellung von RRR-α-Tocopherol.

EP 0 039 830 A1

COMPLETE DOCUMENT

Verfahren zur Herstellung von optisch aktiven Verbindungen und deren Verwendung zur Herstellung von Vitamin E

Vitamin E besitzt 3 Asymmetriezentren und kann demzufolge in 8 stereoisomeren Formen auftreten. Das in der Natur auftretende Stereoisomere der Formel III

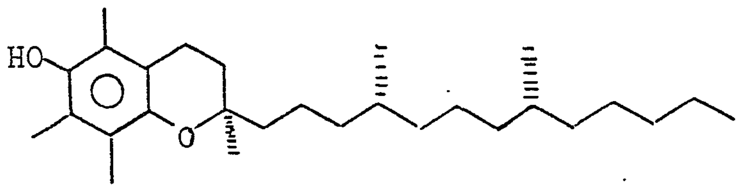

III

besitzt an den 3 Zentren R-Konfiguration.

Die Wirkung der einzelnen Stereosiomeren ist ähnlich, doch besitzt das natürlich vorkommende Stereoisomere die stärkste biologische Wirkung. Zur Darstellung von Vitamin E der oben angegebenen Konfiguration sind verschiedene Verfahren bekannt (z.B. gemäß DE-OS 27 20 775 und 26 02 507), die ausgehend von mikrobiologisch erzeugten chiralen Bausteinen die Seitenkette von III aufbauen. Diese Verfahren liefern zwar eine hohe optische Ausbeute, jedoch unbefriedigende chemische Ausbeuten. Zudem erfordern diese Verfahren aufwendige Trennoperationen zur Isolierung der z.T. wasserlöslichen Zwischenprodukte, die eine technische Durchführung verlustreich und umständlich gestalten. Diese Nachteile werden durch das erfindungsgemäße Verfahren überwunden, das es ermöglicht, zur Synthese von Vitamin E verwendbare bifuntionelle Bausteine der Formel I

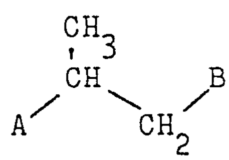

I

in der A eine $-CH_2OR^1$ Gruppe, wobei $R^1$ für Wasserstoff, niedermolekulares Alkyl, Aryl oder niedermolekulares Acyl

steht, ferner eine gegebenenfalls acetalisierte oder acylierte Formylgruppe oder eine Carboxylgruppe bedeutet, B eine gegebenenfalls acetalisierte oder acylierte Formylgruppe oder eine Gruppe der Formel $-COOR^2$ bedeutet, in der $R^2$ für einen niedermolekularen Alkylrest steht, und wobei A und B stets verschieden sind und wobei ferner A und B zusammen die Gruppe $CH_2$-O-CO- bedeuten können, in einfacher Weise in sehr guter chemischer Ausbeute in optisch angereicherter Form so herzustellen, daß man Verbindungen der Formel II

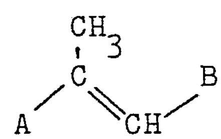

II

in der A und B die oben angegebenen Bedeutungen haben, in Gegenwart eines in der Reaktionsmischung löslichen Komplexkatalysators hydriert, der aus einer Rhodium- oder Rutheniumverbindung und einem chiralen Phosphin- oder Arsin-Liganden gebildet ist.

Beispielsweise kommt ein Komplexkatalysator der Formel Me $X_n$ $L_3$ in Betracht, in der Me Rhodium oder Ruthenium, X Chlor, Brom oder Jod, L einen Phosphin- oder Arsin-Liganden und n die Zahlen 1 bis 3 bedeutet, wobei mindestens einer der Liganden $L_3$ optisch aktiv ist.

Unter einem chiralen Phosphin- oder Arsin-Liganden wird ein Arsin oder vorzugsweise ein Phosphin oder Diphosphin verstanden, in dem mindestens einer der organischen Reste mindestens ein chirales Kohlenstoffatom enthält und/oder in denen mindestens ein Phosphoratom chiral ist.

Die löslichen Komplexe des Rutheniums und vorzugsweise Rhodiums mit dem chiralen Phosphin können entweder außerhalb des Reaktionsmediums oder "in situ" unter den Reaktionsbedingungen erzeugt werden. Die letztgenannte Methode ist die einfachere Arbeitsweise und ist daher bevorzugt.

Als Ausgangs-Rhodiumverbindungen kommen beispielsweise Rhodiumhalogenide und insbesondere Komplexe des Rhodiums mit Olefinen in Betracht, der allgemeinen Formel

$$[Me\ X\ (R)_m]_2$$

in der

Me Rhodium oder Ruthenium,

X ein Halogenatom, z.B. Chlor oder Brom und

m eine ganze Zahl von 1 bis 4 und

R ein aliphatisches oder cycloaliphatisches Olefin oder Diolefin

bedeutet, wie z.B. Ethylen, Propylen, Buten, Isobuten, Butadien, Hexadien-1,5, Hexadien-1,4- Octadien-1,5, Isopren, Cyclohexadien-1,3, Cyclooctadien-1,5.

Als solche Komplexe mit diesen Olefinen sind beispielsweise zu nennen:
$\mu$,$\mu$'-Dichlor-bis-(cyclohexadien-1,3-rhodium) (=CODRhCl), $\mu$,$\mu$'-Dichlor-bis-(cyclooctadien-1,5-rhodium), $\mu$,$\mu$'-Dichlor-bis-(diethylen-rhodium).

Weiterhin sind Carbonylkomplexe der Formel

$$Me\ H(CO)\ (L^1)_3$$

zu nnen, in der Me Rhodium oder Ruthenium, $L^1$ einen einzähligen oder mehrzähligen achiralen Phosphinliganden der Formel $P(R)_3$ bedeutet, wobei der Rest R für die achiralen Reste Alkyl, Cycloalkyl oder Aryl steht wie Methyl, Ethyl, Propyl, Butyl, Pentyl, Hexyl, Octyl, Cyclohexyl, Phenyl

oder Tolyl. $L^1$ ist dabei vorzugsweise Triphenylphosphin.

Als Rhodiumcarbonylkomplexe der genannten Formel kommen im einzelnen Tetrarhodimdodecacarbonyl und Hexarhodiumhexadecacarbonyl in Betracht.

Als chirale Phosphine für die erfindungsgemäß zu verwendenden Katalysatoren können Mono- als auch Diphosphine Verwendung finden.

Chirale Monophosphine sind z.B. Diphenylmenthylphosphin, Phenyldimenthylphosphin und Trimenthylphosphin.

Bevorzugt sind jedoch die chiralen Diphosphine der Formel

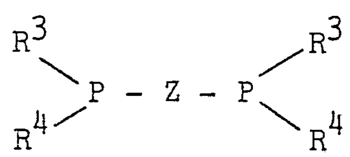

in der $R^3$ und $R^4$ gleiche oder verschiedene Kohlenwasserstoffreste mit 1 bis 15 Kohlenstoffatomen bedeuten. Z bedeutet eine Valenz oder einen organischen, zweiwertigen Rest, wobei mindestens einer der Reste $R^3$, $R^4$ oder Z chiral ist.

Im einzelnen bedeuten $R^3$ und $R^4$, die vorzugsweise identisch sind, Alkylreste mit 1 bis 10 Kohlenstoffatomen, wie Methyl, Ethyl, Isobutyl, sec-Butyl, sec-Pentyl, Ethyl-2--hexyl, Cycloalkylreste mit 4 bis 8 Kohlenstoffatomen, wie Cyclobutyl, 1-Methylcyclobutyl, Cyclohexyl, 1-Methylcyclohexyl, 2-Methylcyclohexyl oder Aryl- oder Aralkylreste wie Phenyl, Naphthyl oder Tolyl.

Der Rest Z steht für

- einen linearen oder verzweigten Alkylenrest mit 1 bis 10 Kohlenstoffatomen, einen gegebenenfalls alkylsubstituierten Cycloalkylrest mit 3 bis 7 Kohlenstoffatomen im Ring, einen Arylenrest oder einen zweiwertigen polycyclischen Rest, wobei diese Reste gegebenenfalls durch einen oder mehrere inerte funktionelle Reste substituiert sein können, insbesondere 1 bis 3 Alkoxygruppen mit 1 bis 4 Kohlenstoffatomen;

- einen zweiwertigen heterocyclischen Rest (wie Pyperidylen oder Dioxa-1,3-cyclopentylen-4,5), der 1 oder 2 Heteroatome wie Sauerstoff oder Stickstoffatome enthält;

- eine Kette aus einem oder mehreren Alkylen- und/oder Cycloalkylen und/oder zweiwertigen heterocyclischen und/oder polycyclischen Rest, wie sie weiter unten im einzelnen angegeben sind;

- eine Verknüpfung über Alkylenreste wie sie vorstehend bereits genannt wurden, jedoch mit tertiären Aminogruppen, die gegebenenfalls direkt an die Phosphoratome gebunden sein können.

Als Beispiele für Z als chiralen divalenten Rest sind Reste der folgenden Formeln zu nennen:

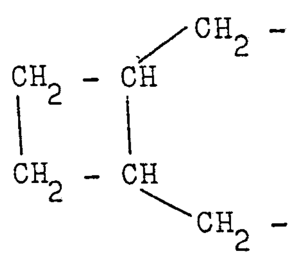

(a)

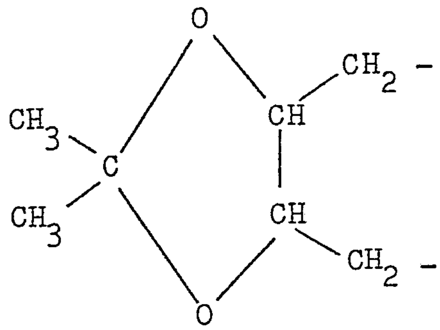

(b)

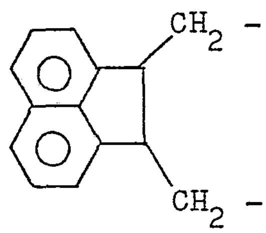

(c)

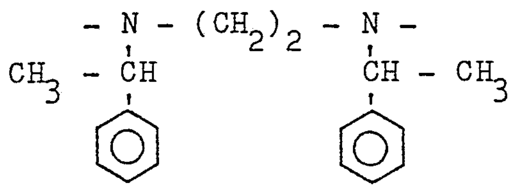

(d)

Unter den chiralen Diphosphinen kommen beispielsweise folgende Verbindungen in Betracht:

1,2-Bis-(diphenylphosphinomethyl)-cyclobutan (DPCB),
1,2-Bis-(dimethylphosphinomethyl)-cyclobutan,
1,2-Bis-(di-n-butylphosphinomethyl)-cyclobutan,
1,2-Bis-(dioctylphosphinomethyl)-cyclobutan,
1,2-Bis-(ditolylphosphinomethyl)-cyclobutan,
1,2-Bis-(dinaphthylphosphinomethyl)-cyclobutan,
1,2-Bis-(ethylhexylphosphinomethyl)-cyclobutan,
1,2-Bis-(diphenylphosphinomethyl)-cyclopentan,
1,2-Bis-(diphenylphosphinomethyl)-cyclohexan,
4,5-Bis-(dimethylphosphinomethyl)-2,2-dimethyl-1,3-dioxolan,
4,5-Bis-(diphenylphosphinomethyl)-2,2-dimethyl-1,3-dioxolan (DIOP),
4,5-Bis-(ditolylphosphinomethyl)-2,2-dimethyl-1,3-dioxolan,

1,2-Bis-(dimethylphosphinomethyl)-acenaphthen,
1,2-Bis-(dibutylphosphinomethyl)-acenaphthen,
1,2-Bis-(diphenylphosphinomethyl)-acenaphthen (DPA),
1,2-Bis-(ditolylphosphinomethyl)-acenaphthen,
1,4-Bis-(diphenylphosphino)-2,3-dimethoxybutan (DDB),
1,2-Bis-(diphenylphosphino)-2-methyl-ethan,
N-Acetyl-4-diphenylphosphino-2-diphenylphosphinomethyl-pyrrolidin,
Tetramenthyldiphosphin und Bis-(N,N'-diphenylphosphino)--bis-[N,N'-(phenyl-1-ethyl)]-diaza-1,4-butan.

Neben dem erwähnten DIOP sind folgende weitere chirale Phosphine bevorzugt:

1,2-Bis-(Diphenylphosphino)-1-phenylethan (DPPE),
2,3-Bis-(Diphenylphosphino)-bicyclo-[2,2,1]-heptan (NORPHOS),
Neomenthyl-diphenylphosphin (NMDP),
2,3-Bis-(Diphenylphosphino)-1,4-dimethoxy-butan (DPB),
Tetramenthyldiphosphin,
Diphenyl-(2-Methylbutyl)-phosphin (DMBP),
2,3-Bis-(diphenphosphino)-propan.

Beispiele von Phosphinen mit chiralem Phosphoratom sind Methylcyclohexyl-o-methoxyphenylphosphin, Methylcyclohexylphenylphosphin, Benzylphenylmethylphosphin.

Wie bereits erwähnt wird der Komplex aus dem Rhodiumderivat und dem chiralen Phosphin in der Regel "in situ" hergestellt. Die dazu verwendete Menge des Phosphins hängt von der Art des Phosphins und der Art des Rhodiumderivates ab. Diese Menge kann, ausgedrückt als Verhältnis Grammatome Phosphor zu Grammatomen Rhodium zwischen 0,5 und 10 schwanken; dieses Verhältnis P/Rh ist vorzugsweise 1 bis 6. Man kann auch höhere Verhältnisse P/Rh als 10 wählen, ohne daß jedoch dadurch ein Vorteil entstünde.

Die zu hydrierenden Verbindungen der Formel II können als E/Z Isomerengemische eingesetzt werden. Zur Erzielung hoher optischer Ausbeuten ist jedoch die Verwendung stereochemisch einheitlicher Substrate vorteilhaft.

Die Verbindungen der Formel II werden mit katalytischen Mengen, z.B. einer Menge von 0,01 bis 10 Molprozent Katalysator in einem inerten Lösungsmittel, wie Essigester, Alkoholen, aromatischen und aliphatischen Kohlenwasserstoffen und Nitrilen unter einem Druck von 1 bis 100 bar, vorzugsweise 1 bis 20 bar hydriert. Die Temperatur der Hydrierung ist nicht kritisch und kann zwischen -20 und +150°C, vorzugsweise zwischen 0 und 100°C gewählt werden. Abhängig von Druck und Temperatur ist das Auftreten von Folgereaktionen; so kann z.B. eine Aldehydgruppe in Verbindung der Formel II bei höherem Druck leichter zum Alkohol reduziert werden als bei niederem. Ein so bei der gleichzeitig erfolgenden Hydrierung der olefinischen Bindung erhaltenes Produkt ist aber ebenfalls zur Überführung in Vit. E geeignet, so daß diese zusätzlich auftretenden Reaktionen die breite Anwendbarkeit des Verfahrens unterstreichen. Die Produkte der Formel I enthalten in Abhängigkeit von den spezifischen Reaktionsbedingungen einen mehr oder weniger großen Überschuß an einem der beiden Enantiomeren. Dabei läßt sich durch geeignete Wahl der rechts- oder links-drehenden Form des chiralen Phosphins im Katalysator sowohl ein Überschuß an rechts- als auch links-drehendem Anteil in den erfindungsgemäß erhältlichen Verbindungen erzeugen, wodurch die Synthese von stereoisomeren Formen des Vitamins E, die wahlweise an den Zentren 4' und 8' anders als in der oben angegebenen Formel III konfiguriert sind, besonders einfach durchführ-

bar wird, im Gegensatz zu den bekannten mikrobiologischen Verfahren, die nur die Herstellung eines einzigen Enantiomeren ermöglichen.

Die Hydrierung kann auch unter Zusatz von Basen wie z.B. Natriummethylat oder Triethylamin durchgeführt werden, wobei eine Zunahme der Reaktionsgeschwindigkeit zu beobachten ist.

Das erfindungsgemäße Verfahren unterscheidet sich von anderen bekannten homogenen Hydrierverfahren, z.B. den Verfahren zur Hydrierung von Itaconsäure (R. Achiwa, Tetrahedron Lett. 1475 (1978)) darin, daß bifunktionelle Substrate der Formel II eingesetzt werden, deren funktionelle Gruppen chemisch unterscheidbar sind. Erst durch diese chemische Unterscheidbarkeit wird eine Verwendung der erhaltenen Produkte der Formel I als Zwischenprodukte zur Synthese von Vitamin E möglich.

Die Überführung der Zwischenprodukte erfindungsgemäß erhältlicher Verbindungen der Formel I in Vitamin E kann in an sich bekannter Weise nach den Angaben von M. Schmid und R. Barner, Helv.Chim.Acta 62, 464 (1979) und R.Zell, Helv. Chim.Acta 62, 474 (1979) erfolgen.

Ausführungsbeispiele

1. Zu einer aus 216 mg /u,/u'-Bis-(1,4-cyclooctadien)-rhodiumchlorid und 865 mg (-) DIOP in 80 ml entgastem Ethanol hergestellten Katalysatorlösung werden 2,00 g 4-Methyl-5-hydroxy-2-oxo-2,5-dihydrofuran in 10 ml Ethanol gegeben und 4 Tage bei Raumtemperatur und 1 bar hydriert. Nach dem Einengen erhält man 5-Ethoxy-4-methyl-2-oxo-2,5-dihydrofuran als Diastereomorengemisch (ca. 70/30): Zur Bestimmung der

optischen Reinheit wird das Rohprodukt in 20 ml THF aufgenommen, mit 1,5 g $LiACH_4$ portionsweise versetzt, die Mischung 3 Stunden bei 50°C gerührt und nach üblicher Hydrolyse mit 1,5 NaOH im Kugelrohr destilliert. Man erhält 0,94 g farbloses Öl, das nach gaschromatographischer Analyse einen Gehalt von 85% an 2-Methyl-pentan-1,5-diol aufweist. $[\alpha]_D^{25}$= +6.51 (= 61% e.e.) (c = 3.49/Methanol).

2. Man gibt eine Lösung von 0,30 g 2-Methyl-4-formyl--(E)-but-2-en-säure und 2 µl Triethylamin in 40 ml absol. Toluol zu einer aus 65,5 mg (-)-NORPHOS, 32,4 mg µ,µ'-Bis-(1,4-Cyclooctadien)-rhodiumchlorid-Dimer und 16 ml Toluol hergestellten Katalysatorlösung. Es wird 20 h bei 5 bar hydriert, filtriert, mit 3 ml Ethanol und 0,19 g $NaBH_4$ 3 h bei Raumtemperatur gerührt, mit Wasser versetzt, mit $CH_2Cl_2$ extrahiert, getrocknet und im Kugelrohr bei 120°C/0,1 Torr destilliert. Man erhält 0,22 g Methylbutyrolacton als farbloses Öl (85 % d.Th.) (Gehalt nach GC: 100 %).

3. Zu 390 mg (−)-DIOP und 250 mg CODRhCl fügt man unter Argon 0,56 g 4-Methyl-2-oxo-2,5-dihydrofuran und 20 ml entgastes Ethanol zu und hydriert bei 2 bar und Raumtemperatur 64 h; darauf wird filtriert, eingeengt und im Kugelrohr bei 130°C/20 Torr destilliert. Man erhält 0,55 g (98 % d.Th.) 3-Methylbutyrolacton als farbloses Öl (Gehalt nach GC: 99 %).

<u>Beispiel 4 bis 18</u>

In analoger Weise wie in Beispiel 2 beschrieben werden die in der Tabelle 1 angegebenen Ergebnisse erhalten.

| Beispiel Nr. | Verbindung II A | B | Verhältnis Z/E | Katalysator | Mol% Rh | Mol% P | Temp. (°C) |
|---|---|---|---|---|---|---|---|
| 4 | $-CH_2-O-\underset{O}{\underset{\|}{C}}-$ | | 100/0 | CODRhCl/(-)-DIOP | 10 | 15 | 0 |
| 5 | " | | 100/0 | " /(+)-DIOP | 2 | 5 | 40 |
| 6 | " | | 100/0 | " /(+)-NORPHOS | 2,5 | 5 | RT |
| 7 | " | | 100/0 | $Rh_4(CO)_{12}$/(-)-DIOP | 2 | 5 | RT |
| 8 | $CO_2H$ | CHO | 0/100 | CODRhCl/(-)-DIOP | 2,5 | 5 | RT |
| 9 | " | " | 0/100 | " /(+)-DIOP | 2,5 | 5 | RT |
| 10 | " | " | 0/100 | " /(+)-NMDP | 1 | 7 | RT |
| 11 | CHO | $CH_2OAc$ | 1/99 | " /(+)-DIOP | 2,5 | 5 | RT |
| 12 | " | " | 1/99 | " /(+)-DMBP | 2 | 5 | RT |
| 13 | $CH_2OH$ | $CO_2Et$ | 1/99 | " /(+)-DIOP | 3 | 5 | RT |
| 14 | $CH(OCH_3)_2$ | $CH_2OAc$ | 9/91 | " /(+)-DIOP | 3 | 5 | RT |
| 15 | CHO | 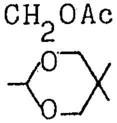 | 2/98 | " /(+)-DIOP | 3 | 5 | RT |
| 16 | $CH_2OAc$ | 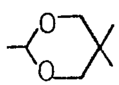 | 25/75 | " /(+)-DIOP | 3 | 5 | RT |
| 17 | $CH_2OH$ | 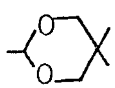 | 27/73 | " /(+)-DIOP | 4 | 9 | RT |
| 18 | CHO | $CO_2Et$ | | " /(-)-NORPHOS | 3 | 5 | 30 |

RT = Raumtemperatur

| Beispiel Nr. | Zeit (h) | Lösungsmittel | Druck $H_2$ (bar) | Verbindung I A | Verbindung I B | Gehalt nach GC |
|---|---|---|---|---|---|---|
| 4 | 15 | Ethanol | 2 | $-CH_2-O-\overset{O}{\overset{\|}{C}}-$ | | 99 |
| 5 | 15 | Methanol | 2 | " | | 99 |
| 6 | 14 | Ethanol | 1 | " | | 100 |
| 7 | 10 | Ethanol | 1 | " | | 99 |
| 8 | 20 | Ethanol | 5 | $CO_2Et$ | $CH_2OH$ | 95 |
| 9 | 20 | Ethanol | 5 | " | " | 90 |
| 10 | 18 | Methanol | 1 | $CO_2CH_3$ | " | 100 |
| 11 | 20 | Toluol | 10 | $CH_2OH$ | $CH_2OAc$ | 85 |
| 12 | 20 | Toluol | 5 | " | " | 90 |
| 13 | 24 | Toluol | 2 | $-CH_2-O-\overset{O}{\overset{\|}{C}}-$ | | 50 |
| 14 | 16 | Ethanol | 1 | $CH(OEt)_2$ | $CH_2OAc$ | 51 |
| 15 | 10 | Toluol | 2 | $CH_2OH$ | 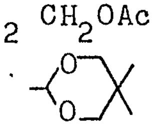 | 75 |
| 16 | 10 | Toluol | 10 | $CH_2OAc$ | 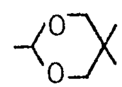 | 15 |
| 17 | 36 | Toluol | 10 | $CH_2OH$ | 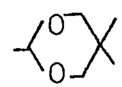 | 95 |
| 18 | 5 | Toluol | 10 | $CH_2OH$ | $CO_2Et$ | 80 |

Et = $C_2H_5$; Ac = $\overset{O}{\overset{\|}{C}}CH_3$

## Patentansprüche

1. Verfahren zur Herstellung von optisch aktiven Verbindungen der Formel I

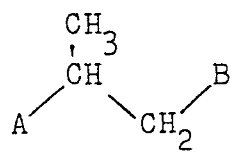

I

in der A eine $-CH_2OR^1$ Gruppe, wobei $R^1$ für Wasserstoff, niedermolekulares Alkyl, Aryl oder niedermolekulares Acyl steht, ferner eine gegebenenfalls acetalisierte oder acylierte Formylgruppe oder eine Carboxylgruppe bedeutet, B eine gegebenenfalls acetalisierte oder acylierte Formylgruppe oder eine Gruppe der Formel $-COOR^2$ bedeutet, in der $R^2$ für einen niedermolekularen Alkylrest steht, und wobei A und B stets verschieden sind und wobei ferner A und B zusammen die Gruppe $CH_2$-O-CO- bedeuten können, dadurch gekennzeichnet, daß man Verbindungen der Formel II

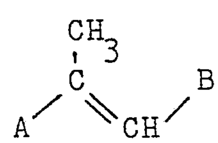

II

in der A und B die oben angegebenen Bedeutungen haben, in Gegenwart eines in der Reaktionsmischung löslichen Komplexkatalysators hydriert, der aus einer Rhodium- oder Rutheniumverbindung und einem chiralen Phosphin- oder Arsin-Liganden gebildet ist.

2. Verfahren gemäß Anspruch 1, <u>dadurch gekennzeichnet</u>, daß man einen Katalysator der Formel

$$MeX_nL_3$$

verwendet, in der Me Rhodium oder Ruthenium, X Chlor, Brom oder Jod, L einen Phosphin- oder Arsin-Liganden und n die Zahlen 1 bis 3 bedeutet, wobei mindestens einer der Liganden $L_3$ optisch aktiv ist.

3. Verfahren gemäß Anspruch 2, <u>dadurch gekennzeichnet</u>, daß als Liganden L optisch aktive Phosphine der Formel

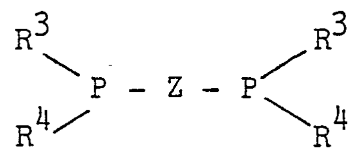

verwendet werden, in der $R^3$ und $R^4$ gleiche oder verschiedene Kohlenwasserstoffreste mit 1 bis 15 Kohlenstoffatomen und Z eine Valenz oder einen organischen zweiwertigen Rest bedeuten, wobei mindestens einer der Reste $R^3$, $R^4$ oder Z chiral ist.

4. Verfahren nach Anspruch 1, <u>dadurch gekennzeichnet</u>, daß man die Komplexe in kationischer Form einsetzt.

5. Verfahren nach Anspruch 2, <u>dadurch gekennzeichnet</u>, daß man Komplexe der Formel

$$MeH(CO)L_3$$

verwendet, in der Me und L die in Anspruch 2 angegebenen Bedeutungen haben.

6. Verwendung der gemäß Anspruch 1 erhältlichen Verbindungen der Formel I zur Herstellung von Vitamin E.

0039830

Nummer der Anmeldung

EP 81 10 3211.9

Europäisches Patentamt

# EUROPÄISCHER RECHERCHENBERICHT

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | TETRAHEDRON LETTERS, Nr. 39, September 1978<br>Oxford<br>T. IKARIYA et al. "Regio-Selective Reaction of Cyclic Anhydride with $RuH_2(PPh_3)_4$"<br>Seiten 3749 bis 3752<br>-- | 1 | C 07 B 1/00<br>B 01 J 31/28<br>C 07 D 311/72<br>C 07 D 307/32<br>C 07 D 307/60 |
| | JOURNAL OF ORGANOMETALLIC CHEMISTRY, Band 188, Nr. 1, 1. April 1980<br>Lausanne<br>M. BIANCHI et al. "Homogeneous Catalytic Hydrogenation of Free Carboxylic Acids in the Presence of Cluster Ruthenium Carbonyl Hydrides"<br>Seiten 109 bis 119<br>-- | 1 | RECHERCHIERTE SACHGEBIETE (Int. Cl.³)<br>B 01 J 31/<br>B 01 J 31/24<br>B 01 J 31/28<br>C 07 B 1/00<br>C 07 D 307/32<br>C 07 D 307/60<br>C 07 D 311/72 |
| | US - A - 4 119 652 (W. S. KNOWLES et al.)<br>* Anspruch 1; Spalte 2, Zeilen 37 bis 51 *<br>-- | 2,3 | |
| P | US - A - 4 220 590 (W.S. KNOWLES et al.)<br>* Anspruch 1 *<br>& DE - A - 2 456 937<br>-- | 3 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | GB - A - 1 332 894 (JOHNSON, MATTHEY & CO.)<br>* Ansprüche 1 bis 15 *<br>-- ./.. | 5 | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 20-08-1981 | KNAACK |

EPA form 1503.1 06.78